# EUROPEAN PATENT SPECIFICATION

(11) **EP 1 841 354 B1**
(45) Date of publication and mention of the grant of the patent: **11.07.2012**
(21) Application number: 06716865.8
(22) Date of filing: 25.01.2006
(51) Int. Cl.: G09B 23/30, A61N 1/362, A61B 19/00, G06F 19/00, A61B 5/03

(54) **A HEART CLUSTER STATE MACHINE SIMULATING THE HEART**
HERZCLUSTERZUSTANDSMASCHINE ZUR HERZSIMULATION
AUTOMATE A ETATS FINIS REGROUPANT PLUSIEURS AUTOMATES ET SIMULANT LE COEUR

(30) Priority: 25.01.2005 SE 0500181
(43) Date of publication of application: 10.10.2007
(73) Proprietor: Gripping Heart AB, 104 25 Stockholm (SE)
(72) Inventor: LUNDBÄCK, Stig, S-185 00 Vaxholm (SE)
(74) Representative: Holmberg, Nils Anders Patrik
(86) International application number: PCT/SE2006/000114
(87) International publication number: WO 2006/080887

(56) References cited:
- WO-A-01/88642
- WO-A1-01/88642
- US-A1- 2004 097 782
- MASLEN E H ET AL: 'Artificial Hearts.' PROCEEDINGS OF THE 1997 IEEE INTERNATIONAL CONFERENCE ON CONTROL APPLICATIONS. 07 October 1997 - 07 October 1997, pages 204 - 209, XP010250901
- STORAA C ET AL: 'Angular Error in Ultrasound Doppler Tissue Velocities and its Influence on the Derived Variable Peak Systolic Strain.' IEEE TRANSACTIONS ON THE 23RD ANNUAL EMBS INTERNATIONAL CONFERENCE. 25 October 2001 - 28 October 2001, pages 131 - 134, XP010593309
- LEWAN M.: 'Gripping Heart: Med full koll pa pumpen.' NY TEKNIK., [Online] 26 May 2005, pages 1 - 4, XP002998737 Retrieved from the Internet: <URL:http://www.nyteknik.se/art/40835>

## Description

### Field of the invention

The present invention relates to a heart cluster state machine describing the pumping and regulating functions of the heart by using databases according to the independent claim. The system is applicable in relation with a large number of various medical investigating methods and devices.

### Background of the invention

It is asserted in the theses Lundbäck S., "Cardiac Pumping and Function of the Ventricular Septum", Stockholm, 1986, that the pumping and regulation of the human heart take place in a manner which is at variance with the prevalent view.
According to the cited publication, the healthy heart performs its pumping action without substantially changing its outer shape and volume.

As a result of the theory presented in the above-mentioned publication regarding the heart's pumping and regulating function a new class of pumps has emerged, a so called dynamic displacement pump or delta (Δ) volume pump (abbreviated as ΔV-pump).

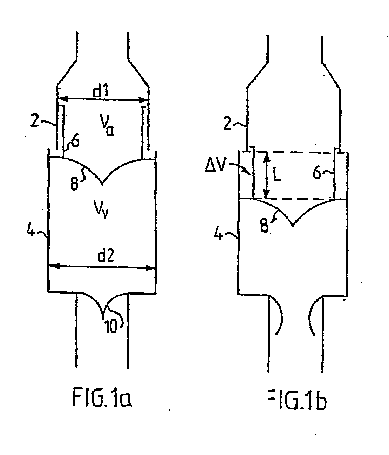

The principles of a ΔV-pump will now be described with references to figures 1a and 1b. The pump comprises an upper cylinder 2 with diameter d1 and a lower cylinder 4 with diameter d2, where d2>d1. These two cylinders are connected to each other via a third cylinder 6 that is freely movably arranged between the upper and lower cylinders. The movable cylinder 6 is provided with a valve 8 at its lowest part that corresponds e.g. to the mitralis valve in the heart. The volume above this valve is defined as the atrial volume (Va) and the volume below the valve is defined as the ventricular volume (Vv). The lower cylinder is provided with an outflow valve 10 at its lowest part that corresponds e.g. to the aortic valve in the heart. As can be seen from figure 1b is a ring-shaped cylindrical volume gradually obtained between the movable cylinder and the inner wall of the lower cylinder when the movable cylinder is moved down, ΔV in the figure. This results in that the volume Va+Vv decreases with the volume ΔV when the movable cylinder moves between its upper position and its lower position.
A source of energy (not shown in the figures) is adapted to move the movable cylinder from its upper position to its lower position, which defines the length L of a stroke for the pump. When the movable cylinder moves down to its lowest position the outflow valve is forced to open and a part of volume Vv is expelled. The movable cylinder is then released from the source of energy and can return to its upper position if there is an inflow to the pump. If Av and Aa designates the cross-sectional areas of the upper and lower cylinder, respectively, ΔV equals L(Av-Aa).

WO-01/88642 relates to a computer based system adapted to create a representation of the pumping action of a heart by using a mathematical model of the functions of the heart based upon the above-described principles of the ΔV-pump in order to make it possible to enhance the methods of analyses, diagnosis and therapy of the heart. The heart is modelled by a computer-based representation of one dynamic displacement pump or of two interconnected dynamic displacement pumps, ΔV-pumps.

Many different requirements, boundary conditions, must generally be met when implementing a mathematical model on to a pump, describing its construction, power source, pumping and regulating functions in a circulatory system. There will be even more boundary conditions if the circulatory system comprises two circulatory systems, as is the case with the heart, and pumps, where the flow to and from the two circulatory systems always shall be in balance.
One object with the present invention is to reproduce the heart's function as a double pump serving two circulatory systems, made and driven by the heart muscle cells, as a mechanical model with the dynamic boundary conditions that the heart has in the body.

An overall object with present invention is to arrange a system or a model for simulating the heart adapted to be used in modern imaging creating and analysing systems that can reproduce working models of a pumping heart in three dimensions with all its functions, sizes and muscular mass with dynamic boundary conditions equal to what the nature has developed.

A reproduced model of the pumping and regulating functions of the heart in an individual specific circulatory system opens up a number of different possibilities. Among those it will in a much better way bring knowledge and understandings of the pumping and regulating functions of the heart, and in cheaper and better ways validated diagnosis, prognosis, medical and surgery treatments (reconstructive heart surgery with artificial and or biological materials) and follow up studies for patients, health care and training athletes.

### Summary of the invention

The above object is achieved by a heart cluster state machine according to the independent claim.

Preferred embodiments are set forth in the dependent claims.

In particular the invention relates to a system related to dynamic boundary conditions stored in preferably relational databases for the heart being a cluster state machine. This cluster state machine is a result of a fusion of dynamic boundary conditions of finite heart muscle cell state machines and dynamic boundary conditions of a ΔV-pump state machine. The newly created machine is a cluster state machine that in the following text will be referred to as the heart cluster state machine or as the ΔV-heart pump.
By the dynamic boundary conditions of the functions of the ΔV-heart pump and other known dynamic boundary conditions, e.g. the boundary conditions of the tissue surrounding the heart, the tonus of the vessels, blood volume, stimulation of the heart etc, a reproduction of the heart's real way of function may be achieved with essentially two main reproduction methods.

Method nr 1 is a three dimensional reproducing method of the heart's function with powerful computer aided support able to handle dynamic boundary conditions in relational databases down to the chemical micro-level of the heart as a ΔV-heart pump. In addition to other known dynamic boundary conditions such as e.g. the surrounding tissues of the heart and vessels, on individual level, this method may achieve a full reproduction of the heart and its dynamic functions in the circulatory system. Since this method is operating and modelling the heart and the circulatory system with natural dynamic boundary conditions down to the chemical and micro-level of the heart muscle cell, it is possible to modulate, simulate, calculate the hearts functions and even mimic the architectures of heart-muscles way of working.

Method nr 2 may be regarded as a lighter version of Method nr 1. Here the dynamic boundary conditions of the ΔV-heart pump are stored as validated data in databases, preferably relational databases, related to one or more logical state diagrams of a the heart being a ΔV-pump. The dynamic boundary conditions of the finite muscle cell state machines are held in the background but are of course, being the origin of in the validated data, represented in the databases. This method cannot give a full detailed reproduction of the heart and its dynamic functions in the circulatory system, but it can serve as databases describing the true pumping and regulating functions of the heart. The databases can either be ideal data obtained from hundreds of individuals at same conditions like sex, age, weight, physical conditions etc. and/or being data from one single individual acquired from e.g. method 1. The database from the single individual can be compared with the databases with ideal data or be compared with the database from the same individual at another time for e.g. comparing the effects of medical treatments, physical training etc.

Both methods may generate individual specific data that will indicate when, where, how and why the heart performs its pumping and regulating functions as it does. This will in a much better way, compared to established techniques, bring knowledge and understandings of the pumping and regulating functions of the heart. It will in less expensive and better ways validate diagnosis, prognosis, medical and surgery treatments (e.g. reconstructive heart surgery with artificial and or biologic materials) and follow up studies for patients, health care and training athletes.

### Short descriptions of the appended drawings

The present invention will now be described in detail with references to the appended drawings.
Figure 1a and 1b schematically illustrates the principles of a ΔV-pump.
Figure 2 is an example of a logical state diagram of the heart being a ΔV-pump.

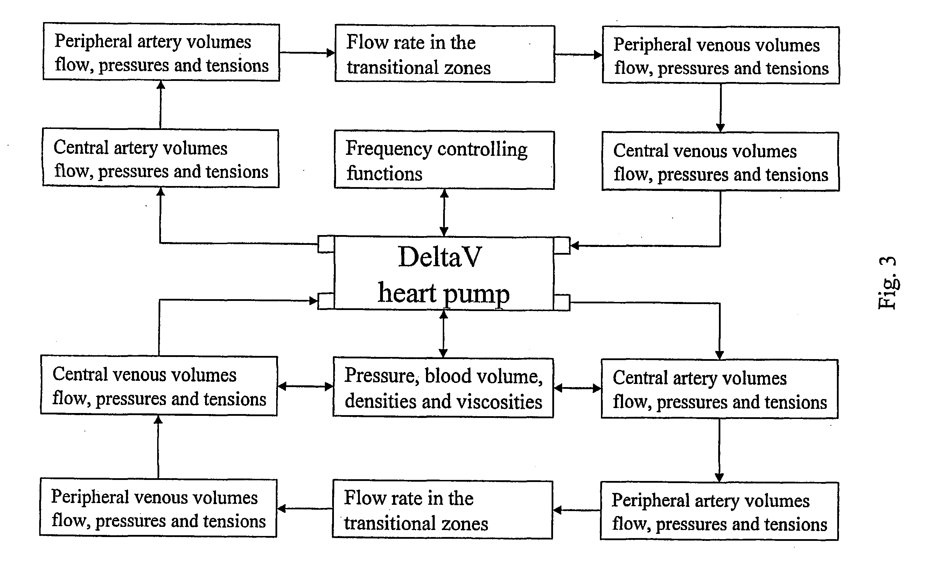

Figure 3 is a block diagram illustrating the computerized model according to the invention showing the main boundary conditions in a circulatory system with dynamic boundary conditions of a ΔV heart pump.
Figure 4 is an example of a block diagram that schematically illustrates the ΔV-heart pump and the circulatory system according to the present invention.

### Detailed description of preferred embodiments of the invention

The key to reproduce the heart and its functions is to define the fundamental boundary conditions that the nature has been able to fulfill, creating the pumping and regulating functions of the heart.

According to the present invention this is achieved by transforming the heart in technical terms to a heart cluster state machine running with the dynamic boundary conditions that normally are set by the nature. The heart cluster state machine for simulating the heart and the circulatory system of an individual is a result of fusions of dynamic boundary conditions of finite heart muscle cell state machines to a muscular network, the heart muscle, adapted to the dynamic boundary conditions of a ΔV-pump state machine. The created heart cluster state machine also being referred to as the ΔV-heart pump, will follow the dynamic boundary conditions of said finite heart muscle cell state machine and of said ΔV-pump state machine.
The working condition of the cluster state machine will be equal to the working conditions of the heart inside a body and may be expressed by databases, preferable relational databases, by using generally available computing, imaging, storage, and analysing systems.

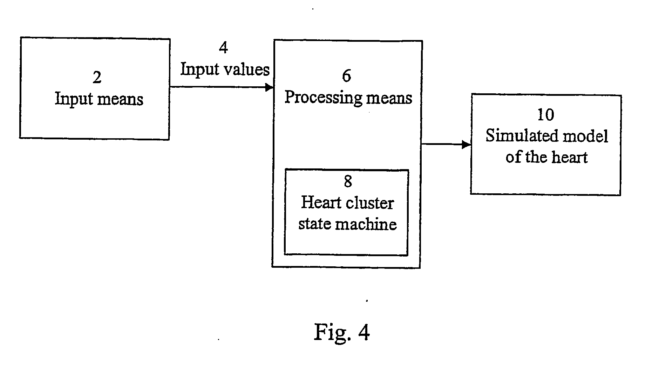

With references to figure 4, a preferred embodiment of the present invention is illustrated. The heart cluster state machine is realized by a system including input means 2 for receiving a set of input values 4 related to the heart and the circulatory system. The received set of values is applied to a processing means 6 that is adapted to generate , by using the set of input values, a relational database system being such that it both satisfies the working regimen of the heart muscle and the working regimen of the ΔV-pump of the heart cluster state machine.

The set of input values may be measured as single or mixed imaging data of the heart obtained by ultrasound, magnetic resonance, x-ray, gamma radiation or by using other imaging or physiological data of the heart such as e.g. ECG, FCG, Apex cardiogram, pulse and/or flow measurements, pressure and/or volume changes over time.

According to another preferred embodiment of the present invention the processing means is adapted to analyse input values by using the predetermined database systems related to the ΔV heart-pump and the circulatory system. Input values may be obtained as single or mixed values by a medical measurement device adapted to detect values related to physiological data over time.
The system may be included in sophisticated imaging devices and in more simple devices as, e.g. blood pressure measuring devices or pulse pressure sensors. It can also be included in internal devices like pacemakers, transponders etc.

In a still further preferred embodiment the relational databases of the system can automatically or in response of command instructions from an operator, be used to correct or change the displayed heart and circulatory system. In this way the system also can serve as a simulating system in order to determine a therapeutic treatment, e.g. surgery or pharmaceutical treatment.

The system can also serve as a mechanical link between different kinds of investigation methods, results and data bases related to the heart and circulatory system. In that way it will be a natural communicating system e.g. on Internet and Telemedicine, between professional users, professional users and related individuals, and by individuals themselves.

Thus, by the present invention it has been achieved to simulate the heart's pumping and regulating functions, with modem technologies, as a state machine that fulfils the dynamic boundary conditions of both heart muscle cell state machines as well as the dynamic boundary conditions of a ΔV-pump state machine. That means that the heart's natural pumping and regulating functions, together with other known dynamic boundary conditions in the natural circulatory system, may be simulated as computerised models, which opens up a wide range of applications.

As briefly discussed above, instead of pumping with squeezing functions being the traditional pumping movement of the heart, the present invention is based upon the observations that the heart is pumping with back and forth going movements with a piston-like unit, referred to as the Delta (Δ) V-piston or the spherical ΔV-plane. The area of the piston consists of a more flat area and a curved area. The flat area consists of the ring of annulus fibrosis, the AV-ring, and its four valves which means that it includes the connection areas of aorta and the pulmonary artery T. Pulmonalis.
The curved area being convex in two-dimensional imaging or spherical in three-dimensional imaging consists of the left and right muscles connected to the flat area, the ring of annulus fibrosis.
When the ΔV -piston is drawn towards the apex of the heart and forces the blood contained in the ventricles into the pulmonary and systemic circulation, it will at the same time draw blood into the atria and its auricles as a consequence of the boundary conditions of the ΔV heart pump. The convex parts, areas, of the ΔV-piston that are in direct contact with the pericardia including the projected areas of Aorta and Pulmonalis that are in direct contact with the surrounding tissues will form the direct ΔV volumes. The areas of the ΔV-piston that are in indirect contact with the surrounding volumes will form the indirect ΔV-volumes. Such areas are mostly covered by the auricles and to a certain extent T. Pulmonale and Aorta.
During the beginning of ventricular diastole, during the phase when the ventricular muscles start to be relaxed, the ΔV-piston starts to return to its the initial position by filling up the ΔV volumes it generated during the contraction of the ventricles. That is done under influence of dynamic and static forces of the masses and by stored energy in the heart structures and its surroundings, created by the downward movement of the ΔV-piston during ventricular systole. The pressure gradients over the ΔV areas generate a hydraulic return of the ΔV-piston, and is referred to as the ΔV-function.
Most of the outer volume changes are the direct and indirect ΔV volumes in connection to the motion of the ΔV-piston. The abilities (as described in the cited theses) of the heart to change the relative volumetric capacities of the right and left ventricles is mainly done by motions of the common ventricular wall, the ventricular septum. During ventricular diastole the relaxed state of the muscles the ventricular septum can adapt its form and position depending of the pressure gradients between the two ventricles. During ventricular systole the ventricular septum together with the rest of the left ventricular heart muscle assumes an essentially cross circular cross-sectional configuration and takes a distinct position independently of its shape and position during diastole. This is so, because during ventricular systole the pressure in the left ventricle is always higher than the pressure in the right ventricle. If the configuration and position of the ventricular septum during diastole, the relaxed state, are different from the configuration and position during systole, the active state, the ventricular septum, acting like a diaphragm pump, therefore provides an increased stroke volume for one ventricle and a correspondingly reduced stroke volume for the other ventricle. In this way, the ventricular septum accomplishes a double-acting regulation to maintain the balance between the two branches of the circulatory system (the pulmonary circulation and the systemic circulation).

The dynamic boundary conditions needed to describe the heart as a ΔV heart pump (heart cluster state machine) are clarified by giving examples of subdivided boundary conditions for the working principles of the muscle cell and subdivided boundary conditions for the working principles of the heart being a ΔV pump.
I The dynamic boundary conditions of a muscle cell as being a finite state machine, can be subdivided in boundary conditions and working principles as follows:
   Ia the boundary conditions of chemical, electrical and mechanical ways of creating power and triggering the finite muscle state machines being parts of a conduction system in order to, in synchronized ways, achieve optimal order for the pumping- and regulating functions of the heart.
   Ib the boundary condition of a connective tissue network around the muscle cells allowing firm constructions, elongating and shortening with enough space for the muscles being thicker at the muscular contraction.
   Ic the boundary conditions of arranging muscle cells to create a four-chamber volume pump acting like a ΔV-pump but serving to circulatory systems keeping them in an exact balance. Naturally, two and three chamber hearts will have other conditions.
II The dynamic boundary conditions of the heart working as a ΔV-pump state machine are subdivided in boundary conditions and working principles as:
   IIa The boundary conditions of surrounding tissues encapsulating a four-chamber volume with in- and outlets having functions and properties supporting the ΔV functions of the heart.
   IIb. The boundary conditions of a movable ΔV-piston, having valves, and outlet vessels, dividing an inner continuous volume of the heart into supplying and expelling volumes and also generating ΔV volumes arranged to create ΔV functions.

   In traditional circulatory systems with ordinary pumps it is usually the speed of the pumps that controls both the inflow and outflow. That is not the case with the Dynamic Displacement pumps, the ΔV-pumps. They are inherently controlled by the inflow. The ΔV-volumes creates ΔV-functions that determine the stroke length and in case of the heart also determine the sizes of the heart as a ΔV-pump. This means that the ΔV heart pump has to be incorporated in a circulatory system to show or create its true pumping and regulating functions. In this way the dynamic boundary conditions controlling the venous return will have a very important role in controlling the cardiac output. The ΔV heart pump will, if the frequency and power are high enough, always try to pump away the blood that is coming through its inlet vessels. This has earlier not been fully understood. The main dynamic boundary conditions of circulatory system that are needed to support or being supported by the ΔV-heart pump can be described as:
III Dynamic boundary conditions of the central venous volumes (e.g. pressure, flow, volumes, tensions of the larger veins including the pulmonary veins leading to the heart).
IV Dynamic boundary conditions of the peripheral venous volumes (e.g. the blood volume exchange and storage capacity of capacitance vessels).
V Dynamic boundary conditions of the central arterial volumes (e.g. pressure, flow, volumes, tensions of the larger arteries including the pulmonary arteries leaving the heart).
VI Dynamic boundary conditions of the peripheral arterial volumes (e.g. the variations of blood volumes needed to support different organs at different times and activity's controlling the flow rate in the transitional zones, and pressure drop to values of the venous pressures).
VII Dynamic boundary conditions of keeping the total blood volume, blood densities and viscosities.
VIII Dynamic boundary conditions for controlling heart rates and blood pressures.

With the heart presented as a ΔV heart pump it will be possible to modulate and simulate the natural circulatory system. The synergies between the functions of the heart and the functions of the circulatory systems will be better understood and will increase the demands of having answers to the questions when, where, how and why the heart does perform as it does. It will for example be very useful in medical treatments, intensive care and research.

In other words and being an essential part of the present invention, each muscle cell must be arranged/configured such that it both fulfils the conditions for its own working regimen and also fulfils the requirements as a part of the structure building up the heart as a ΔV-pump. The working regimen creating power by shortening and thickening and the boundary conditions behind that are well known.

All experimental working models of the heart have under all circumstances been described with squeezing functions. This was obviously the case when the heart was supposed to do its pumping and regulating functions by external squeezing motions of the atria and ventricles in a rhythmic counter acting way. This is still close to 100% believed to be the true pumping functions among ordinary people and doctors in general.
With the new Magnetic Resonance Imaging technique (MRI) the opinion among leading researcher for the fourth time in history adopt the idea that the heart is close to be a constant volume pump pumping with the AV-plane.
It has during at least 200 years been known and generalized that heart muscle is built up by three layers. One outer layer with longitudinally twisted spiral fibres running counter clockwise from the AV-ring down towards Apex were it formats a circular loop and returns to the AV-ring as a clockwise longitudinally twisted spiral fibres. The circular loop formats the inner muscular layer.
Finding the filling forces to the heart has always been a problem. In order to find these forces a few years ago the so called Ventricular Myocardial Band Theory was lounged to solve the mysterious filling of the heart. In that model the outer and inner layers are used to make a counter clockwise and a clockwise rotation of the heart by delayed contractions called a systolic ventricular filling. Thickening of the heart muscle as giving the pumping function. Very recently this theory was totally denied by anatomical specialist that had investigated the heart muscles in thin slices with electron microscopy. They found no layers that could slide against each other. They could verify the earlier known orientations of the muscle fibres and that the left ventricle also had strong circular orientated muscle cells in the centre of the muscle. That was not the case for the right ventricle.

Thus the muscular fibres has an orientation like an "X "with an additional circular "---" fiber orientation in the left ventricle.

These fibre orientations are very suitable to be used in the ΔV-heart pump. This pump concept are only using the outer lining motions for its pumping and regulating functions. That means:
the linings towards the pericardial sac,
the linings and AV-ring with valves separating the inflow volume from the outflow volume,
the lining that taper the area of the interventricular septum towards the right ventricle and the complex but very functional lining that is dividing the right and left atria and auricles from each other and the outlet of aorta and T. Pulmonalis.

The muscles way of working by shortening and thickening will become a matter of doing packing and unpacking in a proper physiologic order. The thicker the muscular wall has to be the harder it will be to solve the task.

With the above-described dynamic boundary conditions millions of vectors will cooperate and build up the ΔV heart pump and its functions having shapes, structures and functions that the real heart in fact has.

According to the present invention a logic state diagram of the heart being a ΔV heart pump with the above mentioned dynamic boundary conditions can be followed and described with practical event markers seen in different kinds of investigation methods. Here is the event markers set following seven main logical states or phases that easily can be seen in Echocardiography. For practical reasons describing the fundamental mechanics behind the ΔV-heart pump concept these event markers are set by events related to the left ventricle. Of course the same events related to the right ventricle should be taken in account in investigating methods where they can be found, though the interaction between the right and left heart is of great importance for the ΔV-heart pump concept. The difference in intensities and timing may serve as good and sharp diagnostic tools. Every change in e.g. timing between these major states will have an impact of the following state and serve as diagnostic tools telling when, where, and why the heart is pumping as it does.

### State 1

### Slow ΔV phase.

This phase was earlier referred to as the slow filling phase. But in this context, where the heart works as a ΔV-pump, the "slow ΔV-phase" is more relevant. It is a direct continuation of the rapid ΔV phase, the returning movement of the ΔV-piston. During slow flow and low rates the slow ΔV phase is relatively long.
During this phase the muscle cells of both the atrias and the ventricles, as well as the ventricular septum, are totally relaxed. The left and right halves of the heart may principally be regarded as common volumes inside the pericardium. This results in that the right and left half of the heart, respectively, forms, together with the incoming vessels, compliance volumes. The energy in the incoming flows to the left and right atria result in that the volume of the heart primarily increases in the vicinity where the ΔV-piston moves. This generates energy to the ΔV-functions resulting in that the ΔV-piston changes its shape and position and also generates stretching forces to the ring of annulus fibrosis. The energy in the incoming flows is transferred to both ventricles essentially without being disturbed by the ventricular septum.
The total volume of the heart is depending on the heart frequency and inflow.
The size of the ΔV-pump will be set during this state.

The pericardium and its environment are the main limitations to the possible volume expansion of the heart. During this phase the static forces in the inflowing blood are the most prominent forces. Those surfaces forming the indirect ΔV-volumes (mostly the auricles of the atria) do not contribute during this phase to any net forces to press the ΔV-piston in the direction to the top of the heart. It is mainly the direct ΔV-volumes formed by the enlargements of the heart in connections to the ΔV-piston and the outgoing vessels that performs that action. The egg-like shape of the heart results in that the net forces and the motion of the ΔV-piston towards the top of the heart are limited. The AV-piston will enter into a neutral balanced position. This will limit the stroke length of the ΔV-piston, but the widening of the ΔV-piston encompasses larger volumes. Thus, the heart as a ΔV-pump adapts its size and form in relation to the incoming flow and heart rate.

The filling pressures of the right and left heart halves, respectively, determine the pressure gradient over the ventricular septum. The pressure gradient determines the shapes and positions of the ventricular septum between the right and left ventricles.

This state and state 2 and 3 form, together with the previous state (which is state 7), the prerequisite for the double regulating function of the ventricular septum.

### State 2

### Atrial systolic phase.

According to established teaching the atrial systolic contraction and its associated ECG-signal was the starting point when describing the heart's pumping function. The time between two atrial contraction was denoted a heart period or a heart cycle.
The discovery that the heart works as a ΔV-pump implies that its pumping and controlling functions are controlled of the incoming flow which in turn implies that a description of a heart cycle must start with the slow ΔV phase. The results of the atrial systolic phase depends upon many different parameters and may under certain circumstances result in that the atrial contractions do not add anything to the heart's pumping function, whereas during other circumstances it gives life-sustaining contribution.

During low rates and reduced momentum behind the ΔV-functions in state 7, the atrial contractions contribute to lift the ΔV-piston above its neutral position in state 1. The atrial contraction is a rapid activity. The hydraulic attachments of the atria and its auricles to the pericardia and to the spherical part of the ΔV piston, create during atrial contractions a withdrawal sliding motion on the top of the relaxed and formable ΔV piston and along the pericardial sac. This will create a hydraulic power that forces the ΔV piston in the direction to the top of the heart. During the contraction there will be a redistribution of the blood volume between the atrias and ventricles at a minimum of external and internal acceleration of masses. The pulling of the ΔV piston to the top of the heart is favoured by quick atrial contractions because then the momentum against motion of the inner and outer masses are large. Since the total volume of the heart is fairly constant during the atrial contraction the sliding motions of the ΔV-piston against the pericardial sac only results in a redistribution of blood between the atria and the ventricles. The more or less only areas that can generate a need of external inflow volumes during atrial systole are the outflow tracts of T-pulmonalis and Aorta. These areas can generate both direct and indirect ΔV-volumes. During atrial contraction there is an inflow to the right atria but usually there is a small backflow from the left atria. This is most likely depending of small compliance volumes in the pulmonary veins and the fact that the left auricle is squeezed between the ΔV- piston and the lung veins and thus widening the veins during a withdrawal contraction. During large flows and high heart rates, with large momentum behind the rapid return of the ΔV-piston, the flow dynamics behind the ΔV-functions force the ΔV-piston to passes its neutral position. The role of the slow ΔV phase bringing the heart to a full size ΔV-pump is reduced, due to large dynamic forces and a background of static forces that can keep the heart at full size. The atrial contraction can more or less not contribute to any further motion of the ΔV-piston to the heart base.

During small ΔV-piston movements, caused by a lot of reasons, low momentum behind the returning motions of the ΔV-piston, phase 6, the atrial contraction can contribute, up to 60%, of the stroke volume by lifting the ΔV-piston to the base of the heart.

The mechanism behind the dramatic differences regarding the importance of the atrial contraction during high and low flows and rates, respectively, and during heart failure, has never had any mechanical explanations before. That is also true for the role that the auricles plays for the pumping function. The heart as a ΔV-pump gives an important mechanical explanation of the atrial contraction and the auricles role for the pumping function.
It also explains why the inflow to the heart can continue despite ongoing atrial contractions.

After atrial systole follows the ventricular systolic expelling phase, here divided in three states. Since the pressure during this phase usually is much higher in the left ventricle, the left ventricle can be looked upon being a separate ΔV-pump working in collaboration with the ΔV heart pump.

### State 3

### Presystolic volume to tension phase

After the atrial contraction the conduction system, after a certain AV-delay, in synchronised orders, starts to depolarise muscle cells in the ventricles. During state 3 (earlier called the iso-volumetric phase) the muscle not only has to create power to the heart but also has to, being the construction material, strengthen the parts of the heart that within the next time interval will be exerted by high forces.
The ventricular septum, the apical and conical parts of the ventricles and the papillary muscles will be activated first. Within a few milliseconds thereafter the initiation is spread to the rest of the heart, that means the spherical muscular sphincter like parts of the ventricles, i.e. the ΔV-piston. The way of activation of the ventricles may be regarded as a "soft start", and is useful during later phases when the ΔV-piston starts its relaxation and returning movements.

The initiation follows a pattern that optimises the presumptions of the ΔV-piston movement towards apex. Interventricular septum starts stabilizing in order to withstand the pressure gradients between the left and right ventricles. The left ventricle format with interventricular septum and its connections to the AV-ring and outflow tract of Aorta, as a direct continuation of its external shape, an internal sector of the ΔV-piston, that will interact with the volumes in the right ventricle.
The started activation of the ventricular heart muscle results in increased tensions in the heart muscles. This results in force vectors that by the construction both want to narrow the gap between the ΔV-piston and the apical- diaphragmal region of the heart and also to generate pressure gradients towards the enclosed blood volumes. The tension will create a motion in the fields where resistant against motion is lowest. The hydraulic attachments of the heart to the pericardia and the surrounding tissues creates, as is the case during the atrial contraction, sliding motions of the ventricular muscles along the pericardial sac due to that the resistance to motion of the inside and outside masses are large. An internal redistribution is obtained of the blood volume between the atria and the ventricles but in the reverse direction, resulting in closing of the valves with virtually no back-flow.
A continuing down pulling of the peripheral area of the ΔV-piston, that has a firm connection to the AV-ring and hydraulic connections to the auricles and the pericardia has a concave form in connections to the muscle mass and the enclosed blood volume. This bended form works like a first class lever and can, by bending and pulling, generate and withstand strong force gradients. Of cause this needs extra strong reinforcements of circular oriented muscular fibres in the left ventricle were the pressure gradients over the ventricular wall is much higher.
It is within this bended area that the volume exchanges per stroke length unit will be greatest and it is also here and at the outflow tract of Aorta and T. Pulmonalis that the direct and indirect ΔV-volumes is generated.

In the beginning of the state the right and left ventricles are regarded as one single volume with communicating volumes to the atria and the inflow vessels. During the pull-down of the ΔV-piston and closing of the valves the pressures inside the ventricles increase. The motions of the ventricular septum now reflect what kinds of relationship there were between the static and the dynamic pressures at each side of the ventricular septum at the end of the atrial contraction, and also how the ventricular muscle is activated.

At the end of state 3 the volume redistributions have made the ΔV-piston, the AV-valves and the ventricular septum and the internal sector of the ΔV piston to start to assume the shapes and tensions they need to withstand the pressure gradients that are generated in reaching the pressures that will start an outflow from the right and left ventricles. During normal circumstances all these adaptations occur, in balance with outer resistance of fast volume changes and also concerning the motion of the ΔV-piston in balance with inner fast volume changes. Most of the inner volume changes as results of the sliding motions of the ΔV-piston are done, by internal redistributions of blood volumes. The inflow to the atria can continue, especially at high flow rates, due to their relaxation especially in the areas where the auricles are covering the convex muscular parts of the ΔV-piston and in the areas around the aortic and pulmonary roots where the auricles are filling up volumes that are difficult to access.

State 3 includes many important event and time markers for the heart being a ΔV-pump and the ventricular septum being a regulator for the flow to the pulmonary and to the main circulatory system. With marking points at different locations of the ventricular septum, it can serve as a large and sensitive pressure membrane sensing the on-going activities giving lot of informations about the performance of the heart and the circulatory system. This event can also be monitored by more simple registration method e.g. Apex cardiogram.

### State 4

### Progressive tension and flow phase.

Phase 4 starts as an index mark with the opening of the aortic valve and ends as a marker on top of the aortic outflow. During this phase the motion of the ΔV-piston generates a progressive tension and flow out and into the heart. The pressure is normally much higher in the left ventricle. This results in that the ventricular septum mainly assumes the same shapes as the other parts of the left ventricle. If the systolic shapes and positions deviate from the shapes and positions before the ventricular contractions, a volume adaptation takes place between the ventricles.
As a direct continuation of state 3 the spherical ΔV-piston will create both direct and indirect ΔV-volumes. These volumes, due to external resistance and recoiling forces and increasing blood pressure inside these volumes, will give a net increase of the pressure gradients over the areas producing the ΔV-volumes.

The acceleration of a mass demands power and energy. The masses to be accelerated comprise all tissues in direct and indirect connections to the motion of the ΔV-piston.
These tissues are, all blood in the heart and in the vessels entering or leaving the heart, the heart muscle itself and the masses in the heart's environment. Furthermore, energy must be added for internal and external tension and recoiling forces, and friction losses, as for example created by motions of the Aorta and T. Pulmonalis and twisting torsions of the heart.

During phase 4 larger counter-directed forces are required in order to pull the ΔV-piston towards apex. Due to that and the hydraulic attachments of the heart to the pericardial sac that in turn is hydraulically attached to the chest wall, an increased up-movement takes place of the conical part of the ventricular cylinder in parallel with the chest wall. The phenomena can be mimicked with a vacuum cup that can slide on a slippery surface with forces parallel to the surface but give a high resistance to right angel forces.

The nature has fixated the pericardial sac with strong connected tissues to the diaphragm muscle but not to sternum, were the sac more or less is fixated by a hydraulic coupling. This arrangement avoid problems concerning the breathing mechanism.

The fixation of the pericardial sac, in this way renders the apical diaphragma region of the pericardial sac to act as a resilient suspension that results in a bending and lifting of Apex and the diaphragm against the chest wall. This suspension will more or less take care of all the counteracting forces that the ΔV-piston creates. During the acceleration of the masses in this phase the counteracting forces will reach its highest level. When the accelerations, generated by the muscle contractions, but effected in motions of the ΔV-piston, are over, the suspension will match the resistance and recoiling counteracting forces. Most of the counteracting resistant and recoiling forces are generated outside the common ΔV-piston by the creation of the ΔV-volumes and pulling and twisting the Aorta and T. Pulmonale. The counteracting forces between the ΔV-piston and the diaphragm area wants to separate these areas in both directions. These events and energy will be regained to the pumping functions in the following phases.

By performing measurements during this phase with even simple methods or devices like pulse pletysmography e.g Apex cardiogram and referring these data to the heart being a ΔV heart pump will in many cases give enough information about the hearts pumping and regulating functions within a specific circulatory system.

### State 5

### Regressive flow and tension phase

This phase is in a direct continuation of phase 4 and ends as a marker with the closing of the aortic valve. During this phase both flow and tension starts to decline in the left ventricle that can be looked upon being a separate ΔV-pump working in collaboration with the ΔV heart pump. After phase 4 the declining movements of the ΔV-piston starts. The ΔV-volumes will still be formatted though the indirect ΔV-volumes can be refilled by inflow to the atria and auricles. The twisting of the Aorta and Pulmonalis continue as long as there will be a net motion along the thoracic cage in the direction towards apex. The flow out through the Aorta continues as long as there is a common muscular contraction that can withstand the pressure gradients over the left ventricular walls that can be done by a first liver of function in the muscular part of the ΔV-piston. This part of the ΔV-piston and the diaphragm part of the left ventricle has external forces that together with the pressure inside the ventricle want to separate these areas from each other.
During phase 4 and 5 the counter-directed forces above the ΔV-piston decline. The reasons for that are partly that the acceleration of the masses has stopped and partly that the compliance volumes in the incoming veins to the atria and the indirect ΔV-volumes especially located in the auricles have started to be refilled. The ventricles, looked as solid units, can start, because of stronger recoiling forces in the diaframal area, to return to the neutral position this area had before phase 3. Due to the mechanical coupling this returning movement also results in a relative movement of the ΔV-piston, giving possibilities for continuous inflow into the atrial volumes despite that the real movement between the ΔV-piston and the tip of the cone declines and stops. In addition there is a declining pressure and flow in Aorta and in T. Pulmonalis which result in that their diameters decrease which in turn through their contact to the atria and auricles give room for continuous inflow into the atrial cylinder. The relative movement, but also the real movement of the ΔV-piston, is most pronounced in the region of the outflow tract of T. Pulmonalis.
The ongoing inflow above the ΔV-piston and the decreasing outflow from the heart will cross each other during this phase, which means that the heart will have its smallest total volume before the end of ventricular systole.
By performing measurements during this phase with even simple methods like pulse pletysmographs e. g. Apex cardiogram and referring these data to the heart being a ΔV heart pump will in many cases give enough information about the heart's pumping and regulating functions within a specific circulatory system.
This phase stops for practical reasons with the closing of the aorta valves but is in a middle of an ongoing process, further described under state 6.

### State 6

Prediastolic tension to volume phase
This phase was earlier called the isovolumetric diastolic phase.

This phase has a mechanical action that is running in a reverse way compared to state 3. That means that in order to release the pressure gradients in this described region, the left ventricle, there has to be an increase of the left ventricular volume. That can be done without disturbing any ongoing inlet flow to the heart and at higher heart rates and minute volumes also leave possibilities for ongoing outlet flow. The ongoing process in phase 5 with decreasing pressure gradients to the surroundings of the heart are, as earlier described, concentrated to the muscular parts of the ΔV-piston and the outflow tract of Aorta and T.Pulmonalis. Furthermore, these areas together with the areas in close connections to the diaphragm, which happens to be a part of the left ventricle, have contracting recoiling forces that want to separate these areas from each other through elongation and sliding motions of the ventricular walls along the thoracic cage. This surface of the heart also describes the longest distance between the ΔV-piston and Apex and has a very strong convex attachment of the ventricular muscles to the AV-ring and the sharp bend of T. pulmonalis. This part of the ΔV-piston is well covered by the left and right auricles and need a strong support of muscle power. When that support goes down, the two areas, the ΔV-piston and diaphragm area, start to be separated. This will both lead to a decrease in tension leading to internal redistributions of volumes and finally open the mitral- and tricuspide valves. This event can also be monitored by more simple registration method e.g. Apex cardiogram.

### State 7

### Rapid ΔV-phase.

The rapid diastolic returning movement of the ΔV-piston is a direct continuation of phase 6. An adapted relaxation means that stored energy in the surroundings, the twisting of the heart, can be released in a way that in optimal ways can bring the ΔV-piston back towards the top of the heart. The adapted relaxation creates a total release of the recoiling forces that wanted to separate the total ΔV-piston from the diaphragm area. This will add energy to the inflowing blood in the direction towards apex. Static and dynamic forces of the inflowing blood will exert a pressure on the areas that has created the ΔV-volumes, that means the ΔV-piston, and will create, by moving the ΔV-piston, a refilling of those areas. The movement of the ΔV-piston also creates a redistribution of blood between the auricles, atrias and the ventricles and also in an early stage between the ventricles by a forth and back going motion of the interventricular septum. The enhanced dynamic forces in the directions to apex will be reversed by the ΔV-volumes that finally absorb the static and dynamic forces by filling the direct and indirect ΔV-volumes pressing the ΔV-piston towards the top of the heart. This action is referred to as the ΔV-function and will give the ΔV-piston a rapid diastolic return and dynamic forces behind the valves that together with the flow paradox will close the valves with no back flow. The return of the ΔV-piston will result in a thinning out of the left ventricular muscle, a motion that inside the heart will look like an internal peristaltic expansion wave front running from the ΔV-piston towards Apex.

This event can also be monitored by more simple registration method e.g. Apex cardiogram.

At low frequencies the ΔV-piston performs an overshoot and a recoiling movement. This is an effect of the forces of inertia that the blood has acquired and stored in an expanding wave behind the valves pushing the ΔV-piston in the direction of the direct and indirect ΔV-volumes. Once the dynamic forces have ceased the static forces will dominate and bring the ΔV-piston to a neutral expanding position, state 1.

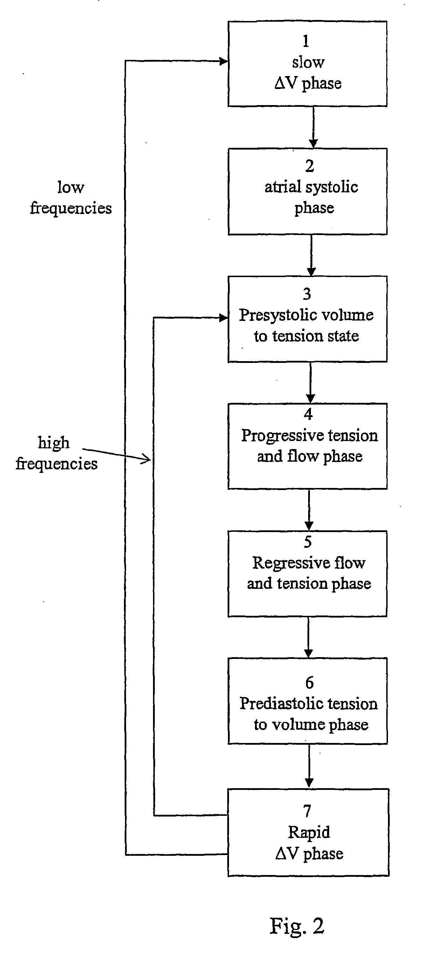

At higher flows and frequencies the slow ΔV phase (state 1), the atrial systolic phase (state 2) and to a certain degree also a part of the early part of the presystolic volume to tension phase (state 3) in flow dynamics point of way will be overruled. The fast diastolic return of the ΔV-piston carried by an expanding wave with a lot of dynamic energy is followed more or less directly by the ventricular contraction (state 3). This is schematically illustrated in the state diagram of figure 2.
The strong expanding wave and the force of inertia will bring the ΔV-piston even higher up to the heart top than the atrial systole can do.

At high flow rates and frequencies ΔV-pumps due to the inertia of the in and outgoing fluid including the fluid in the pump will start to generate a more or less continuous outflow with no need of outlet valves. Still the inlet flow will create the ΔV-functions. The ΔV-pumps start to increase their stroke volumes above that can be calculated by the piston area times the stroke length.
These circumstances applied on the ΔV heart pump will during high inflow rate and high frequencies due to both static and dynamic forces in the blood flow keep the volumes of the heart above the ΔV-piston in a more or less full size at the time when the rapid ΔV phase starts. The volumes of the heart below the ΔV-piston will at the same time be low because the outflow inertia. This will create an increase of the ejection fraction that earlier never has been understood.

Figure 4 is a block diagram that schematically illustrates the ΔV heart pump and the circulatory system according to the present invention. The block diagram illustrates how different physical parameters in the circulatory system are related to each other. The ΔV heart pump in the centre of the figure is provided with two outflow vessels and two inflow vessels whereas the left part of the ΔV heart pump represents the right side of the heart and vice versa. Starting with the lower right outflow vessel in the figure being the aorta, moving clockwise to the lower left inflow vessel represent the vena cava inferior and superior. The upper left outflow vessel being T. pulmonalis and the upper right inflow vessel being the inflow of the lung veins to the left atrium.

The above-mentioned methods will be further discussed in the following.

### Method 1

The complexity of reproducing the heart's functions in a natural circulatory system of a human being or an animal can be reduced to a minimum by creating relational databases that can be within reach of hardware and well known software of today. That is done e.g. according to figure 4 with a processing means 6 that is adapted to generate and handle dynamic boundary conditions of a ΔV heart pump as described above.

The enormous processing and storage capacity of today's computers makes it possible to build huge data banks, to build and/or receive two to three dimensional models and perform computer aided automatic analysing, detections of structures, motions etc. It is also easy with visually instructions like ROI (readings of interest) to give operator instructions directly on the display unit.

There are many well-known techniques and software programs that can create these opportunities, but preferably they need relational databases for their operations. According to the present invention it is now possible, with dynamic boundary conditions and state diagrams of the ΔV heart pump, and dynamic boundary conditions generated by a circulatory system to create dynamic databases, that by processing means automatically and/or with an interactive control by an operator and/or mixed investigating methods may reconstruct the heart and its true functions and flow dynamics. The dynamic databases may also include the dynamic boundary conditions on the chemical micro levels in the heart muscle cells and other cells that have impacts on the circulatory system. In this way method nr 1 can modulate and simulate the whole circulatory system and be used to gain information regarding e.g. when, where, how and why certain medical treatments are effecting the heart and the circulatory system.

The processing means, may in fact, by using databases based upon dynamic boundary conditions of a ΔV heart pump and dynamic boundary conditions of a related circulatory system, generate a three dimensional pumping heart with all its functions, size and muscular mass, just by setting for example, the in- and outgoing flow, pressures and frequency. The output of these input data will present a pumping heart based upon complementary normal dynamic boundary conditions of the ΔV heart pump and other dynamic conditions expressed in databases. Any change of the dynamic boundary conditions may be visualized as a change in constructions and performance of the pumping and regulating functions of the heart.
Other input data may be images obtained from conventional medical imaging systems, e.g. X-ray, ultrasound, MRI etc. The operator or a software program may identify different parts of the heart e.g. the pericardial sac with its surroundings, the diaphragm and chest wall, the atrial and ventricular linings, the ventricular septum, the heart wall, the ΔV-piston, the AV-ring and valves, the conical part of the ventricular cylinder, the auricles and the large vessels. These parts do not need to be whole structures. It can be points, areas or volumes from various sites of the heart not necessary measured with the same investigation method. In certain sites mixed investigating techniques will strengthen the input data as for example the pressure and flow characteristics in the inlet an outlet vessels of the heart. By definition all input data should satisfy the dynamic boundary conditions of the ΔV-heart pump. This means that method 1 also can be used to bridge over the deviations from the true pumping and regulating functions of the heart, that many circulatory investigation methods have today.

The processing means can start with almost any input data generating a three dimensional pumping heart with all its functions, flow and pressure profiles, size and muscular mass. It can even tell if an input data most likely is false. It will use, what is set to be normal boundary conditions, as long as these are not changed. The input data can be generated from mixed investigating methods and with or without manually interference be linked together by the processing means to sharpen the computerized model of the heart and its functions in the circulatory system of the investigated individual.
The ECG-signal is a good coordinator and also a good indicator for well-functioning muscle cells and trigging pathways, but is not always necessary in order to depict the pumping and regulating functions of the heart.

### Method 2

Once the dynamic boundary conditions controlling the ΔV heart pump and the boundary conditions of the related circulation system are known these data can be stored in databases as validated data related to one or more logical state diagrams of a the heart being a ΔV pump. The stored data can either be ideal data obtained from hundreds of individuals at same conditions like sex, age, weight, physical conditions etc. It can also be validated data from one single individual acquired from method 1. New input data can be compared with the ideal databases or be compared with databases from the same individual at another time for e.g. comparing the effects of medical treatments, physical training etc. The computational capacity of the processing means 6 when realizing method 2 may be low as compared with method 1.

The processing means in method 2 can together with a cheap input means, e.g. a small pressure sensor that can generate flow and or pressure profiles, create small cheap but advanced investigating units. These may be integrated in watches, telephones, blood pressure monitoring units, etc. and may, by comparing the input data with related data in the databases, produce output values that are good enough to control and even give diagnostic functions of the heart and the circulatory system for the actual individual. This method can also be used in less powerful stimulating models of the heart and circulatory system in external devices, and in implanted devices like e.g. pacemakers. In this way the cardiac performance stroke by stroke can be checked and corrected e.g. by comparing input values of flow and pressure profiles with ideal values. The compared results can modify the output of ignition power, time and ignition orders at various ignition areas etc, to correct and optimise the pumping and regulating functions of the heart.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A system adapted to realize a heart cluster state machine simulating the heart, and optionally the circulatory system, of an individual, where the heart cluster state machine is achieved by fusions of finite heart muscle cell state machines to form a ΔV-pump state machine and that the state machines are defined by boundary conditions of said heart muscle cell state machines and of said ΔV-pump state machine, and that said heart cluster state machine is adapted to work in accordance with boundary conditions of the surrounding tissue, and inlet and outlet vessels, to the heart, wherein said heart cluster state machine is serving one or two closed circulatory systems having boundary conditions generated in those systems, wherein said system includes;
a processing means (6); and
input means (2) adapted to receive a set of input values (4) related to the heart and the circulatory system, and to apply said set of values to said processing means, wherein said processing means (6) is adapted to generate and handle dynamic boundary conditions of said heart cluster state machine, and to determine, by using said set of input values, a relational database system being such that it both satisfies a working regimen of the heart muscle cell state machines and a working regimen of the ΔV-pump state machine of said heart cluster state machine.

2. System according to claim 1, wherein said processing means is adapted to analyse further input values by using said determined relational database system.

3. System according to claim 1, wherein said system further comprising a display means and that the system is adapted to display said simulated heart and circulatory system on said display means.

4. A blood pressure measuring device comprising the system according to any of claims 1-3.

5. A pulse pressure sensor comprising the system according to any of claims 1-3.

6. An implantable device comprising the system according to any of claims 1-3, wherein said implantable device is preferably an implantable heart stimulator.

## Patentansprüche

1. System, das eingerichtet ist, eine Herzclusterzustandsmaschine zur Herzsimulation, und optional zur Kreislaufsystemsimulation, eines Individuums umzusetzen, wobei die Herzclusterzustandsmaschine durch Zusammenschlüsse von finiten Herzmuskelzellenzustandsmaschinen erreicht wird, um eine ΔV-Pumpenzustandsmaschine auszubilden, und die Zustandsmaschinen durch Randbedingungen der Herzmuskelzellenzustandsmaschinen und der ΔV-Pumpenzustandsmaschine definiert sind, und die Herzclusterzustandsmaschine angepasst ist, in Übereinstimmung mit Randbedingungen des umgebenden Gewebes, und Einlass- und Auslassgefäßen zu dem Herzen, zu arbeiten, wobei die Herzclusterzustandsmaschine ein oder zwei geschlossene Kreislaufsysteme bedient, die in diesen Systemen erzeugte Randbedingungen aufweisen, wobei das System aufweist:
ein Verarbeitungsmittel (6); und ein Eingabemittel (2), das angepasst ist, einen mit dem Herzen und dem Kreislaufsystem verbundenen Satz Eingangswerte (4) zu empfangen, und den Satz Werte mit dem Verarbeitungsmittel zu verwenden, wobei das Verarbeitungsmittel (6) angepasst ist, dynamische Randbedingungen der Herzclusterzustandsmaschine zu erzeugen und handzuhaben, und durch Verwenden des Satzes Eingangswerte eine relationale Datenbank zu bestimmen, die sowohl ein Arbeitsregime der Herzmuskelzellenzustandsmaschinen als auch ein Arbeitsregime der ΔV-Pumpenzustandsmaschine der Herzclusterzustandsmaschine erfüllt.

2. System nach Anspruch 1, bei dem das Arbeitsmittel angepasst ist, weitere Eingangswerte durch Verwenden des bestimmten relationalen Datenbanksystems zu analysieren.

3. System nach Anspruch 1, bei dem das System des Weiteren ein Anzeigemittel aufweist und das System angepasst ist, das simulierte Herz und Kreislaufsystem mit dem Anzeigemittel anzuzeigen.

4. Blutdruckmessgerät mit dem System nach einem der Ansprüche 1-3.

5. Druckpulssensor mit dem System nach einem der Ansprüche 1-3.

6. Implantierbare Einrichtung mit dem System nach einem der Ansprüche 1-3, wobei die implantierbare Einrichtung bevorzugt ein implantierbarer Herzstimulator ist.

## Revendications

1. Système conçu de façon à réaliser une machine à états de groupement du coeur, simulant le coeur et, de façon optionnelle, le système circulatoire, d'un individu, la machine à états de groupement du coeur étant réalisée par des fusions de machines à états de cellules de muscle cardiaque finis de façon à former une machine à états de pompe ΔV, et les machines à états étant définies par des conditions de limite desdites machines à états de cellules de muscle cardiaque et de ladite machine à états de pompe ΔV, et ladite machine à états de groupement du coeur étant conçue de façon à travailler selon des conditions de limite du tissu environnant, et de vaisseaux d'entrée et de sortie, vers le coeur, ladite machine à états de groupement du coeur desservant un ou deux systèmes circulatoires fermés ayant des conditions de limite générées dans ces systèmes, ledit système comprenant :
des moyens de traitement (6) ; et
des moyens d'entrée (2) conçus de façon à recevoir un jeu de valeurs d'entrée (4) associé au coeur et au système circulatoire, et à appliquer ledit jeu de valeurs auxdits moyens de traitement, dans lequel
lesdits moyens de traitement (6) sont conçus de façon à générer et à gérer des conditions de limite dynamiques de ladite machine à états de groupement du coeur, et à déterminer, à l'aide dudit jeu de valeurs d'entrée, un système de base de données relationnelles qui est tel qu'il satisfait tout à la fois un régime de travail des machines à états de cellules de muscle cardiaque et un régime de travail de la machine à états de pompe ΔV de ladite machine à états de groupement du coeur.

2. Système selon la revendication 1, dans lequel lesdits moyens de traitement sont conçus de façon à analyser d'autres valeurs d'entrée à l'aide dudit système de base de données relationnelles déterminé.

3. Système selon la revendication 1, ledit système comprenant de plus des moyens d'affichage, et le système étant conçu de façon à afficher ledit système cardiaque et circulatoire simulé sur lesdits moyens d'affichage.

4. Dispositif de mesure de pression sanguine comprenant le système selon l'une quelconque des revendications 1 à 3.

5. Capteur de pression d'impulsion comprenant le système selon l'une quelconque des revendications 1 à 3.

6. Dispositif implantable comprenant le système selon l'une quelconque des revendications 1 à 3, ledit dispositif implantable étant de préférence un stimulateur cardiaque pouvant être implanté.
